# EUROPÄISCHE PATENTSCHRIFT

(11) **EP 4 393 931 B1**
(45) Veröffentlichungstag und Bekanntmachung des Hinweises auf die Patenterteilung: **02.07.2025**
(21) Anmeldenummer: 22216763.7
(22) Anmeldetag: 27.12.2022
(51) Int. Cl.: C07F 9/6574, C07B 41/06, C07C 45/50, C07C 47/02, B01J 31/18, B01J 31/22

(54) **DIPHOSPHITE MIT DICYCLOHEXYLPHOSPHINOREST**
DIPHOSPHITES WITH DICYCLOHEXYLPHOSPHINOREST
DIPHOSPHITES PRESENTANT DES RESIDUS DE DICYCLOHEXYLPHOSPHINO

(43) Veröffentlichungstag der Anmeldung: 03.07.2024
(73) Patentinhaber: Evonik Oxeno GmbH & Co. KG, 45772 Marl (DE)
(72) Erfinder: SALE, Anna Chiara, 45657 Recklinghausen (DE); FRANKE, Robert, 45772 Marl (DE); FRIDAG, Dirk, 45721 Haltern am See (DE); KUCMIERCZYK, Peter, 44628 Herne (DE); MARKOVIC, Ana, 45721 Haltern am See (DE); BÖRNER, Armin, 18059 Rostock (DE); SELENT, Detlef, 18059 Rostock (DE)
(74) Vertreter: Evonik Patent Association

(56) Entgegenhaltungen:
- EP-A1- 4 074 720

## Beschreibung

Die Erfindung betrifft Diphosphite mit Dicyclohexylphosphinorest und deren Verwendung in der Hydroformylierung.

Phosphorhaltige Verbindungen spielen als Liganden in einer Vielzahl von Reaktionen eine entscheidende Rolle, z.B. in der Hydrierung, in der Hydrocyanierung und auch in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in R. Franke, D. Selent, A. Börner, "Applied Hydroformylation", Chem. Rev., 2012 (112), 11, 5675-5732, DOI:10.1021/cr3001803.

In EP 4 074 720 A1 wird ein Gemisch von Bisphosphiten mit einem offenen und einem geschlossenen Flügelbaustein beschrieben. Die Bisphosphite werden als Katalysatoren in der Hydroformylierung eingesetzt.

Die technische Aufgabe der Erfindung ist die Bereitstellung einer Verbindung, mit welcher bei der Hydroformylierung von Olefinen eine gesteigerte Ausbeute an Aldehyd erzielt werden kann.

Die Aufgabe wird gelöst durch eine Verbindung gemäß Anspruch 1.

Verbindung gemäß Formel (**I**): 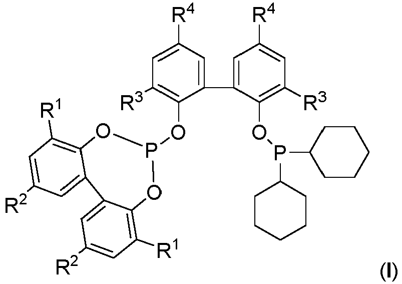 wobei R¹, R², R³, R⁴ für -(C₁-C₁₂)-Alkyl stehen.

In einer Ausführungsform steht R¹ für -^{*ter*t}Bu.

In einer Ausführungsform steht R² für -*^{tert}*Bu.

In einer Ausführungsform steht R³ für -*^{tert}*Bu.

In einer Ausführungsform steht R⁴ für -*^{tert}*Bu.

In einer Ausführungsform weist die Verbindung die Struktur (2) auf:

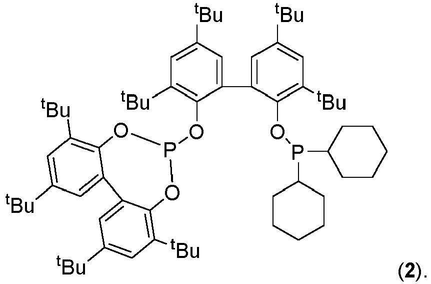

Neben der Verbindung selbst, wird auch ein Verfahren beansprucht, in welchem die zuvor beschriebenen Verbindungen zum Einsatz kommen.

Verfahren umfassend die Verfahrensschritte:
a) Vorlegen eines Olefins;
b) Zugabe einer zuvor beschriebenen Verbindung;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

In einer Variante des Verfahrens ist die Rh-Verbindung ausgewählt aus: Rh(acac)(CO)₂, [(acac)Rh(COD)] (Umicore, acac = Acetylacetonat-Anion; COD = 1,5-Cyclooctadien), Rh₄CO₁₂.

In einer Variante des Verfahrens ist die Rh-Verbindung Rh(acac)(COD).

In einer Variante des Verfahrens erfolgt das Zuführen von H₂ und CO in Verfahrensschritt d) mit einem Druck in dem Bereich von 1 bis 6 MPa (10 bis 60 bar).

In einer Variante des Verfahrens erfolgt das Zuführen von H₂ und CO in Verfahrensschritt d) mit einem Druck in dem Bereich von 1,5 bis 4,5 MPa (15 bis 45 bar).

In einer Variante des Verfahrens erfolgt das Erwärmen des Reaktionsgemisches in Verfahrensschritt e) auf eine Temperatur in dem Bereich von 80 °C bis 160 °C.

In einer Variante des Verfahrens erfolgt das Erwärmen des Reaktionsgemisches in Verfahrensschritt e) auf eine Temperatur in dem Bereich von 100 °C bis 140 °C.

Im Folgenden soll die Erfindung anhand von Ausführungsbeispielen näher erläutert werden.

### Synthese (1): 4,8-Di-tert-butyl-6-((3,3'-di-tert-butyl-2'-((dicyclohexylphosphanyl)oxy)-5,5'-dimethoxy-[1,1'-biphenyl]-2-yl)oxy)-2,10-dimethoxydibenzo[d,f][1,3,2]dioxaphosphepin

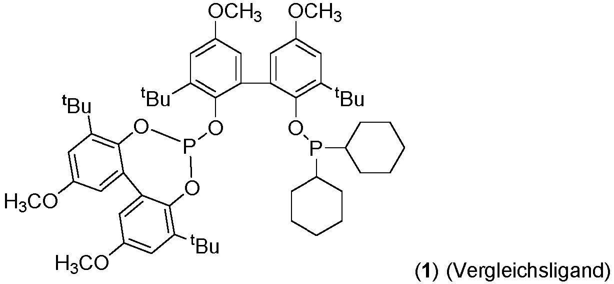

Zu einer Lösung von 3,3'-Di-tert-butyl-2'-((4,8-di-tert-butyl-2,10-dimethoxydibenzo [d,f][1,3,2]dioxaphosphepin-6-yl)oxy)-5,5'-dimethoxy-[1,1'-biphenyl]-2-ol (0,6749 g; 0,906 mmol) in THF (9 ml) wird bei -20 °C tropfenweise eine 0,533 M Lösung von n-Butyllithium (1,7 ml; 0,906 mmol) in Hexan gegeben. Man rührt 20 min bei dieser Temperatur, lässt auf 0 °C erwärmen und tropft eine Lösung von Chlorodicyclohexylphosphin (0,2214 g; 0,9513 mmol) in THF (4 ml) zu. Dann lässt man auf Raumtemperatur erwärmen, rührt über Nacht und entfernt das Lösungsmittel im Vakuum. Der Rückstand wird in Toluol (9 ml) aufgenommen, die entstehende Mischung filtriert, das Filtrat im Vakuum eingeengt und 2 h bei 50°C/0,1 mbar getrocknet. Der erhaltene Rückstand wird aus heißem Acetonitril (8 ml) kristallisiert. Filtration und Trocknen im Vakuum ergibt 0,630 g (0,670 mmol, 74%).

Elementaranalyse (berechnet für C₅₆H₇₈O₈P₂ = 941,18 g/mol): C = 71,35 (71,47); H = 8,40 (8,35); P = 6,42 (6,58)%.

ESI-TOF HRMS: m/z= 941,5244; [M++H], berechnet m/z= 941,5250.

### Synthese (2): 2,4,8,10-Tetra-tert-butyl-6-((3,3',5,5'-tetra-tert-butyl-2'-((dicyclohexylphosphanyl)oxy)-[1,1'-biphenyl]-2-yl)oxy)dibenzo[d,f][1,3,21dioxaphosphepin

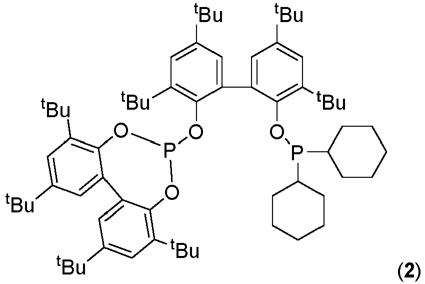

Zu einer Lösung von 3,3',5,5'-Tetra-tert-butyl-2'-((2,4,8,10-tetra-tert-butyldibenzo [d,f][1,3,2]dioxaphosphepin-6-yl)oxy)-[1,1'-biphenyl]-2-ol (0,4897 g; 0,5766 mmol) in THF (5 ml) wird bei -20 °C tropfenweise eine 0,533 M Lösung von n-Butyllithium (1,14 ml; 0,6054 mmol) in Hexan gegeben. Man rührt 20 min bei dieser Temperatur, lässt auf 0 °C erwärmen und tropft eine Lösung von Chlorodicyclohexylphosphin (0,1436 g; 0,6170 mmol) in THF (3 ml) zu. Dann lässt man auf Raumtemperatur erwärmen, rührt über Nacht und entfernt das Lösungsmittel im Vakuum. Der Rückstand wird in Toluol (9 ml) aufgenommen, die entstehende Mischung filtriert, das Filtrat im Vakuum eingeengt und 2 h bei 50°C/0,1 mbar getrocknet. Der Rückstand wird in THF (3,5 ml) gelöst. Der nach Zugabe von Acetonitril (ca. 5 ml) abgeschiedene Feststoff wird filtriert, mit wenig Acetonitril gewaschen und getrocknet. Ausbeute: 0,272 g (0,260 mmol, 45%).

Elementaranalyse (berechnet für C₆₈H₁₀₂O₄P₂ = 1045,50 g/mol): C = 78,23 (78,12); H = 9,74 (9,83); P = 5,71 (5,93)%.

ESI-TOF HRMS: m/z=1045,7322; [M++H], berechnet m/z=1045,7332.

### Synthese (3): ((3,3',5,5'-Tetra-tert-butyl-[1,1'-biphenyl]-2,2'-diyl)bis(oxy))bis (dicyclohexylphosphan)

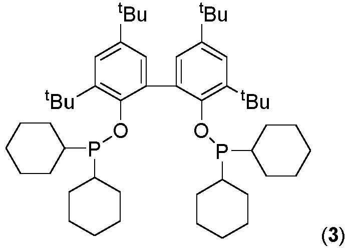

Zu einer Lösung von 3,3',5,5'-Tetra-tert-butyl-[1,1'-biphenyl]-2,2'-diol (0,6832 g; 1,6638 mmol) in THF (12 ml) wird bei -20 °C tropfenweise eine 0,533 M Lösung von n-Butyllithium (6,56 ml; 3,494 mmol) in Hexan gegeben. Man rührt 20 min bei dieser Temperatur, lässt auf 0 °C erwärmen und tropft eine Lösung von Chlorodicyclohexylphosphin (0,8287 g; 3,5606 mmol) in THF (6 ml) zu. Dann lässt man auf Raumtemperatur erwärmen, rührt über Nacht und entfernt das Lösungsmittel im Vakuum. Der Rückstand wird in Toluol (20 ml) aufgenommen, die entstehende Mischung filtriert, das Filtrat im Vakuum eingeengt und 2 h bei 50°C/0,1 mbar getrocknet. Der Rückstand wird in THF (6 ml) gelöst. Der nach Zugabe von Acetonitril (4 ml) abgeschiedene Feststoff wird filtriert und getrocknet. Ausbeute: 0,469 g (0,582 mmol, 35%).

ESI-TOF HRMS: m/z=803,6021; [M++H]; berechnet m/z=803,6025.

### Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol in einem Pure Solv. MD-7 System gereinigt und unter Argon aufbewahrt. Das als Substrat eingesetzten Olefin cis/trans-2-Penten (Aldrich) wurden über Natrium am Rückfluss erhitzt und unter Argon destilliert. Im Autoklaven wurden unter Argonatmosphäre Lösungen der Katalysatorvorstufe und des Liganden, jeweils in Toluol, gemischt. Als Katalysatorvorstufe kam [(acac)Rh(COD)] (Umicore, acac = Acetylacetonat-Anion; COD = 1,5-Cyclooctadien), zum Einsatz. Der Autoklav wurde bei 12 bar für den Enddruck von 20 bar unter Rühren (1500 U/min) aufgeheizt. Nach Erreichen der Reaktionstemperatur wurde das Olefin mit einem in der Druckpipette eingestellten Überdruck in den Autoklaven gepresst. Die Reaktion wurde bei konstantem Druck (20 bar) (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. 1 ml der Reaktionsmischungen wurden unmittelbar nach Abschalten des Rührers entnommen, mit 10 ml Pentan verdünnt und gaschromatographisch analysiert: HP 5890 Series II plus, PONA, 50 m x 0,2 mm x 0,5 µm.

Der Versuch wurde mit den Verbindungen **(1), (2)** und **(3)** durchgeführt.

Die Verbindungen **(2)** und **(3)** dient hierbei als Vergleichsligand.

### Ergebnisse der Katalyseversuche

[Rh]: 100 ppm, p: 20 bar, T: 120 °C; t: 4 h; Rh:L = 1:2

**Tabelle: Hydroformylierung von cis/trans-2-Penten**

| Ligand | Ausbeute Aldehyd [%] |
|---|---|
| **(1)** | 94 |
| **(2)*** | 99 |
| **(3)** | < 1 |

| | |
|---|---|
| * erfindungsgemäße Verbindung | |

Die durchgeführten Versuche belegen, dass die gestellte Aufgabe durch eine erfindungsgemäße Verbindung gelöst wird.

## Patentansprüche

1. Verbindung gemäß Formel (**I):** 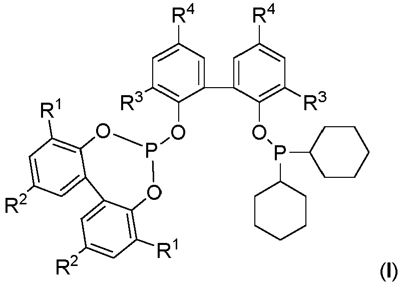 wobei R¹, R², R³, R⁴ für -(C₁-C₁₂)-Alkyl stehen.

2. Verbindung nach Anspruch 1,
wobei R¹ für -*^{tert}*Bu steht.

3. Verbindung nach einem der Ansprüche 1 oder 2,
wobei R² für -*^{tert}*Bu steht.

4. Verbindung nach einem der Ansprüche 1 bis 3,
wobei R³ für -*^{tert}*Bu steht.

5. Verbindung nach einem der Ansprüche 1 bis 4,
wobei R⁴ für -*^{tert}*Bu steht.

6. Verbindung nach einem der Ansprüche 1 bis 5,
wobei die Verbindung die Struktur **(2)** aufweist:

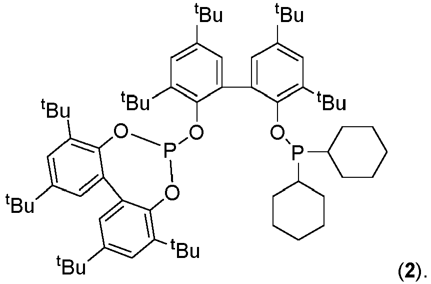

7. Verfahren umfassend die Verfahrensschritte:
a) Vorlegen eines Olefins;
b) Zugabe einer Verbindung nach einem der Ansprüche 1 bis 6;
c) Zugabe einer Rh-Verbindung;
d) Zuführen von H₂ und CO;
e) Erwärmen des Reaktionsgemisches aus a) bis d), wobei das Olefin zu einem Aldehyd umgesetzt wird.

8. Verfahren nach Anspruch 7,
wobei die Rh-Verbindung ausgewählt ist aus: Rh(acac)(CO)₂, [(acac)Rh(COD)] (Umicore, acac = Acetylacetonat-Anion; COD = 1,5-Cyclooctadien), Rh₄CO₁₂.

9. Verfahren nach einem der Ansprüche 7 oder 8,
wobei die Rh-Verbindung Rh(acac)(COD) ist.

## Claims

1. Compound of formula (I): 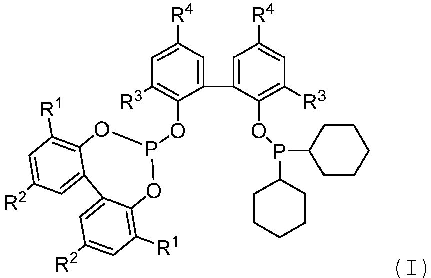 where R¹, R², R³, R⁴ are - (C₁-C₁₂) -aryl.

2. Compound according to Claim 1,
wherein R¹ is -*^{tert}*Bu.

3. Compound according to either of Claims 1 and 2,
wherein R² is -*^{tert}*Bu.

4. Compound according to any of Claims 1 to 3,
wherein R³ is -*^{tert}*Bu.

5. Compound according to any of Claims 1 to 4,
wherein R⁴ is -*^{tert}*Bu.

6. Compound according to any of Claims 1 to 5,
wherein the compound has the structure (2):

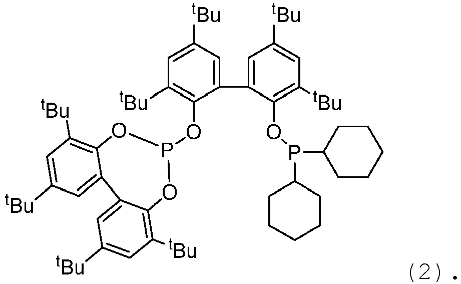

7. Process comprising the process steps of:
a) initially charging an olefin;
b) adding a compound according to any of Claims 1 to 6;
c) adding a Rh compound;
d) feeding in H₂ and CO;
e) heating the reaction mixture from a) to d), to convert the olefin to an aldehyde.

8. Process according to Claim 7,
wherein the Rh compound is selected from: Rh(acac) (CO)₂, [(acac) Rh(COD)] (Umicore, acac = acetylacetonate anion, COD = 1,5-cyclooctadiene), Rh₄CO₁₂.

9. Process according to either of Claims 7 and 8,
wherein the Rh compound is Rh(acac) (COD).

## Revendications

1. Composé selon la formule (I) : 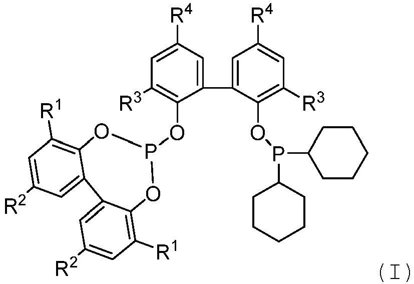 dans laquelle R¹, R², R³, R⁴ représentent -alkyle(C₁-C₁₂).

2. Composé selon la revendication 1,
dans lequel R¹ représente -tert-Bu.

3. Composé selon l'une quelconque des revendications 1 ou 2,
dans lequel R² représente -tert-Bu.

4. Composé selon l'une quelconque des revendications 1 à 3,
dans lequel R³ représente -tert-Bu.

5. Composé selon l'une quelconque des revendications 1 à 4,
dans lequel R⁴ représente -tert-Bu.

6. Composé selon l'une quelconque des revendications 1 à 5,
le composé présentant la structure (2) :

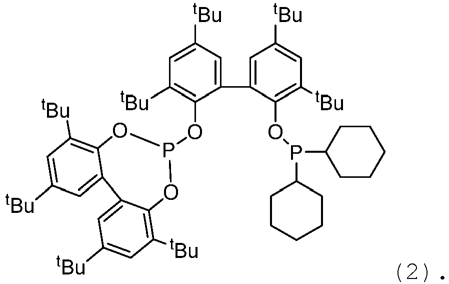

7. Procédé comprenant les étapes de processus :
a) disposition préalable d'une oléfine ;
b) addition d'un composé selon l'une quelconque des revendications 1 à 6 ;
c) addition d'un composé de Rh ;
d) introduction de H₂ et CO ;
f) chauffage du mélange réactionnel provenant de a) à d), l'oléfine étant convertie en un aldéhyde.

8. Procédé selon la revendication 7,
dans lequel le composé de Rh est choisi parmi : Rh(acac) (CO₂, [(acac)Rh(COD)] (Umicore, acac = anion acétylacétonate ; COD = 1,5-cyclooctadiène), Rh₄CO₁₂.

9. Procédé selon l'une quelconque des revendications 7 et 8,
dans lequel le composé de Rh est Rh(acac) (COD).
